# FASCICULE DE BREVET EUROPEEN

(11) **EP 1 263 359 B1**
(45) Date de publication et mention de la délivrance du brevet: **10.08.2005**
(21) Numéro de dépôt: 01911832.2
(22) Date de dépôt: 02.03.2001
(51) Int. Cl.: A61F 5/00

(54) **DISPOSITIF INTRA-GASTRIQUE POUR LE TRAITEMENT DE L'OBESITE MORBIDE**
INTRAGASTRALE VORRICHTUNG ZUR BEHANDLUNG DER MORBIDEN FETTSUCHT
INTRAGASTRIC DEVICE FOR TREATING MORBID OBESITY

(30) Priorité: 13.03.2000 FR 0003161
(43) Date de publication de la demande: 11.12.2002
(73) Titulaire: Districlass Medical S.A., 42000 Saint-Etienne (FR)
(72) Inventeur: WAZNE, Hussein, F-26260 Saint-Bardoux (FR)
(74) Mandataire: Schmitt, John
(86) Numéro de dépôt international: PCT/FR2001/000627
(87) Numéro de publication internationale: WO 2001/068007

(56) Documents cités:
- WO-A-99/25418
- US-A- 4 246 893
- US-A- 5 084 061
- US-A- 5 234 454
- US-A- 5 259 399

## Description

La présente invention est relative à un dispositif intra-gastrique dont la mise en place par voie endoscopique permet le traitement des patients atteints d'obésité morbide.

On connaît des dispositifs permettant de traiter les obésités morbides qui sont constitués d'un anneau dont la mise en place par voie chirurgicale autour du cardia permet de limiter la quantité des aliments absorbés.

La mise en place de ce genre de dispositif est obtenue lors d'opérations chirurgicales lourdes pour le patient.

En outre, ce dispositif comporte certains inconvénients, car il existe de nombreux problèmes ou complications chirurgicales après l'utilisation de cette technique de traitement.

On connaît d'après le brevet US 5 259 399 un dispositif à ballon élastique qui est introduit dans l'estomac par voie externe au moyen d'un trocart permanent fixé sur le patient pour venir déboucher dans l'estomac.

Le dispositif à ballon à volume variable et élastique comporte des moyens d'étanchéité et de fixation sur le trocart pour empêcher toute fuite externe de l'estomac.

On note que ce dispositif comporte certains inconvénients en ce qui concerne son efficacité pour le traitement des obésités morbides.

L'utilisation d'un trocart rigide est pour le patient un handicap majeur car il empêche les mouvements des différents tissus et muscles entraînant des douleurs ou gènes pour le patient.

En outre le ballon élastique à volume variable peut être gonflé jusqu'à obtenir l'occlusion de l'estomac du patient entraînant un risque majeur.

Le ballon à volume variable du fait de son élasticité et lorsqu'il n'est pas gonflé à son volume maximum ne permet pas de s'opposer au volume d'aliments ingérés.

En effet le passage des aliments entraîne une déformation du ballon élastique.

Le dispositif intra-gastrique suivant la présente invention est de mise en place facile par voie endoscopique, qui peut être réalisée par des gastro-entérologues ou des chirurgiens, sous anesthésie locale ou générale.

Ainsi, le dispositif intra-gastrique suivant la présente invention est issu d'un jeu de dispositif comportant chacun un ballon ou enveloppe à volume nominal déterminé.

Ainsi, le dispositif intra-gastrique suivant la présente invention a pour but de remplir un volume par l'intermédiaire d'un ballon à volume nominal déterminé, qui est gonflé à l'intérieur de l'estomac.

Le dispositif intra-gastrique suivant la présente invention permet de réduire le volume de l'estomac, donc la quantité ingérée pour donner une sensation de satiété au patient, sans traumatisme psychologique et physiologique lié à un régime alimentaire strict.

De plus, le ballon du dispositif intra-gastrique suivant la présente invention est retenu contre la paroi interne de l'estomac, offrant un confort pour le patient, et évitant les migrations éventuelles ou le bouchage du tube digestif.

Le dispositif intra-gastrique introduit par voie endoscopique dans l'estomac d'un patient pour le traitement des obésités morbides suivant la présente invention est issu d'un jeu de dispositif comportant chacun un ballon ou enveloppe présentant un volume nominal déterminé qui est raccordé de manière étanche à des moyens de connexion constitués d'un disque formant une base d'appui au ballon contre la paroi interne de l'estomac, d'un tube ou cathéter souple pour le raccordement du ballon à un dispositif d'alimentation, et des moyens d'accrochage solidaires du tube ou cathéter, lesdits moyens de connexion permettant d'une part la mise en place et/ou la récupération du ballon, et d'autre part la fixation, soit à l'extérieur du corps du patient, soit en sous cutané du dispositif d'alimentation pour pouvoir amener le ballon ou l'enveloppe à son volume nominal déterminé.

Le dispositif intra-gastrique suivant la présente invention comprend un disque qui est positionné autour du tube ou cathéter souple et contre la paroi externe du ballon ou enveloppe.

Le dispositif intra-gastrique suivant la présente invention comprend un ballon qui est fixé autour du tube ou cathéter afin que ce dernier débouche à l'intérieur dudit ballon.

Le dispositif intra-gastrique suivant la présente invention comprend des moyens d'accrochage qui sont constitués d'un embout à profil conique solidaire d'un fil.

Le dispositif intra-gastrique suivant la présente invention comprend un embout comportant un tenon qui pénètre à l'intérieur d'un alésage interne du tube ou cathéter.

Le dispositif intra-gastrique suivant la présente invention comprend un fil présentant un profil droit ou en boucle.

Le dispositif intra-gastrique suivant la présente invention comprend un disque qui est de forme circulaire.

Le dispositif intra-gastrique suivant la présente invention comprend un disque qui est de forme ovale.

Le dispositif intra-gastrique suivant la présente invention comprend un ballon ou enveloppe qui présente un profil ovale ou autre qui est symétrique par rapport à l'axe horizontal du tube ou cathéter.

Le dispositif intra-gastrique suivant la présente invention comprend un ballon ou enveloppe qui présente un profil ovale ou autre qui est dissymétrique par rapport à l'axe horizontal du tube ou cathéter.

Le dispositif intra-gastrique suivant la présente invention comprend un ballon ou enveloppe qui présente un profil en forme de haricot symétrique par rapport à l'axe horizontal du tube ou cathéter.

Le dispositif intra-gastrique suivant la présente invention comprend un ballon ou enveloppe qui présente un profil en forme de haricot dissymétrique par rapport à l'axe horizontal du tube ou cathéter.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue illustrant le dispositif intra-gastrique suivant la présente invention.
Figure 2 est une vue suivant F de figure 1 représentant la forme du disque d'appui du dispositif intra-gastrique suivant la présente invention.
Figure 3 est une vue montrant un dispositif d'alimentation du type valve anti-retour qui vient se fixer sur le tube ou cathéter souple du dispositif intra-gastrique suivant la présente invention.
Figure 4 est une vue représentant un autre dispositif d'alimentation du type chambre implantable qui vient se fixer sur le tube ou cathéter souple du dispositif intra-gastrique suivant la présente invention.
Figure 5 est une vue montrant une variante du ballon ou enveloppe du dispositif intra-gastrique suivant la présente invention.
Figure 6 est une vue montrant une autre variante du ballon ou enveloppe du dispositif intra-gastrique suivant la présente invention.
Figure 7 est une vue schématique illustrant la mise en place sur le patient du dispositif intra-gastrique solidaire du dispositif d'alimentation du type valve anti-retour.
Figure 8 est une vue schématique montrant la mise en place sur le patient du dispositif intra-gastrique solidaire du dispositif d'alimentation du type chambre implantable.

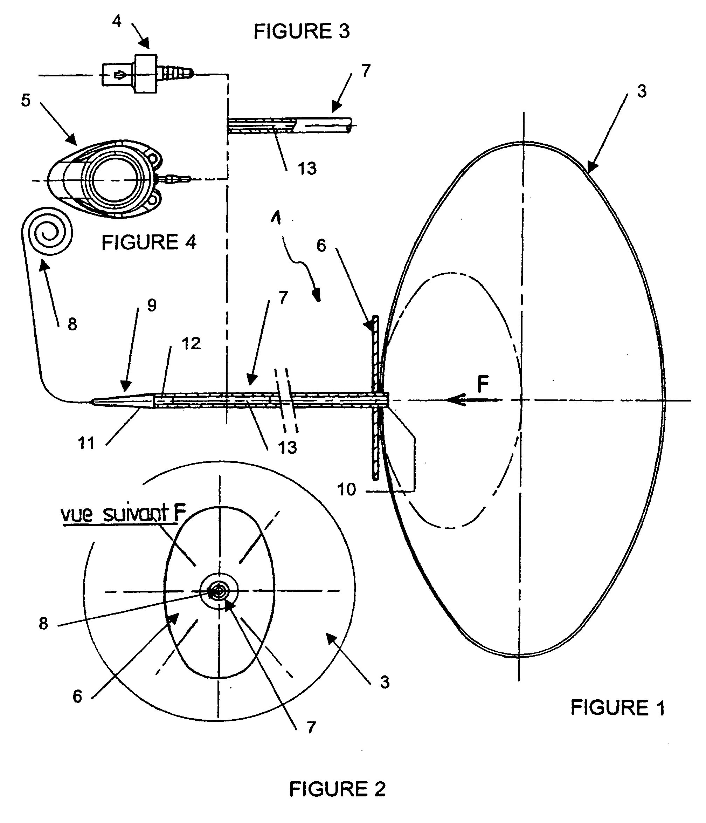

On a montré en figures 1 et 2 un dispositif intra-gastrique 1 implanté dans l'estomac 2 d'un patient, et dont la forme et le volume nominal d'un ballon ou enveloppe 3 est déterminé et permet le traitement d'un patient atteint d'obésité morbide.

Chaque dispositif intra-gastrique est issu d'un jeu de dispositif comportant un ballon ou enveloppe 3 de volume nominal déterminé compris entre 100 (c.c.) centimètres cubes et 1100 (c.c.)centimètres cubes.

Le dispositif intra-gastrique 1 comporte un ballon ou enveloppe 3 de forme ovale ou autre, dont le volume nominal déterminé est obtenu par la quantité de liquide ou de gaz qui est injectée à l'intérieur.

Le dispositif intra-gastrique 1 comporte des moyens de connexion qui permettent, d'une part la mise en place et/ou la récupération du ballon ou enveloppe 3, et d'autre part la fixation, soit à l'extérieur du corps du patient, soit en sous cutané d'un dispositif d'alimentation 4, 5 pour pouvoir amener le ballon ou l'enveloppe 3 à son volume nominal déterminé.

Les moyens de connexion sont constitués d'un disque 6 formant une base d'appui au ballon ou à l'enveloppe 3 contre la paroi interne de l'estomac 2, d'un tube ou cathéter creux 7 qui est souple pour le raccordement à un dispositif d'alimentation 4, 5.

Le ballon ou enveloppe 3 présente un profil ovale ou autre qui est symétrique par rapport à l'axe horizontal du tube ou cathéter souple 7.

En variante le ballon ou enveloppe 3 peut présenter un profil ovale ou autre qui est dissymétrique par rapport à l'axe horizontal du tube ou cathéter souple 7.

Le tube ou cathéter 7 comprend des moyens d'accrochage 9 qui sont disposés à l'opposé du disque 6.

Le disque 6, et le tube ou cathéter 7 sont obtenus dans une même matière plastique, telle que du polyuréthanne ou du silicone ou analogue.

Le disque 6 présente, de manière non limitative, soit une forme circulaire, soit une forme ovale qui s'étend perpendiculairement autour de la périphérie externe du tube ou cathéter 7.

Le disque 6 est positionné autour du tube ou cathéter 7, afin de déterminer une portée cylindrique 10 de faible dimension permettant de constituer un appui sur la paroi interne de l'estomac.

Le disque 6 est positionné autour du tube ou cathéter souple 7 et contre la paroi externe du ballon ou enveloppe 3.

Le disque 6 est conformé de manière à pouvoir subir des déformations élastiques pour venir se plaquer contre la paroi interne de l'estomac 2 lors de la mise en place du dispositif intra-gastrique 1.

Les moyens d'accrochage 9 sont constitués d'un embout 11 à profil conique solidaire d'un tenon 12 qui pénètre à l'intérieur de l'alésage interne 13 du tube ou cathéter souple 7.

Le profil conique de l'embout 11 est prévu pour faciliter la dilatation des tissus, afin d'extraire le tube ou cathéter 7 à l'extérieur de l'estomac 2 et de la paroi abdominale.

L'embout 11 est solidaire dans le prolongement de son profil conique d'un fil 8 qui peut être droit ou en boucle.

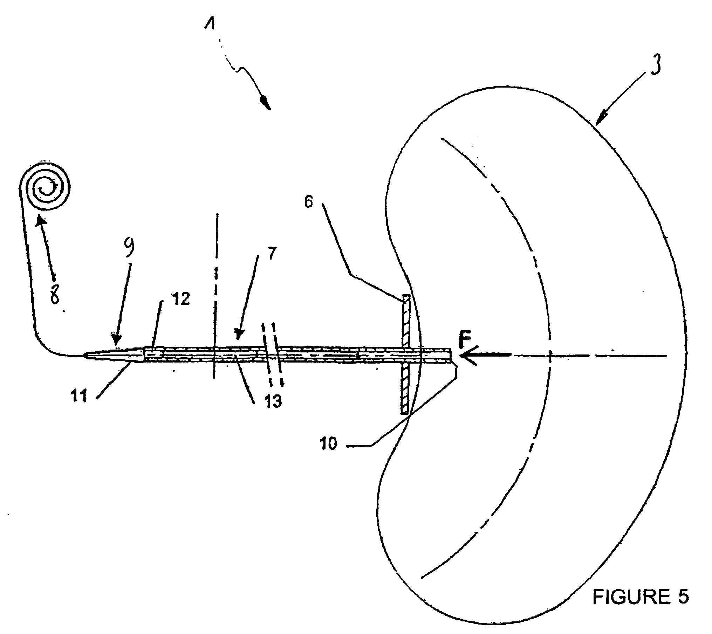

En figure 5 on a représenté le dispositif intra-gastrique 1 suivant la présente invention comprenant un ballon ou enveloppe 3 dont la forme extérieur présente un profil en haricot qui est symétrique par rapport à l'axe horizontal du tube ou cathéter souple 7.

Ce profil particulier du ballon ou enveloppe 3 permet lorsque ce dernier est amené à son volume nominal déterminé à une meilleure adaptation au contour interne de l'estomac 2 du patient.

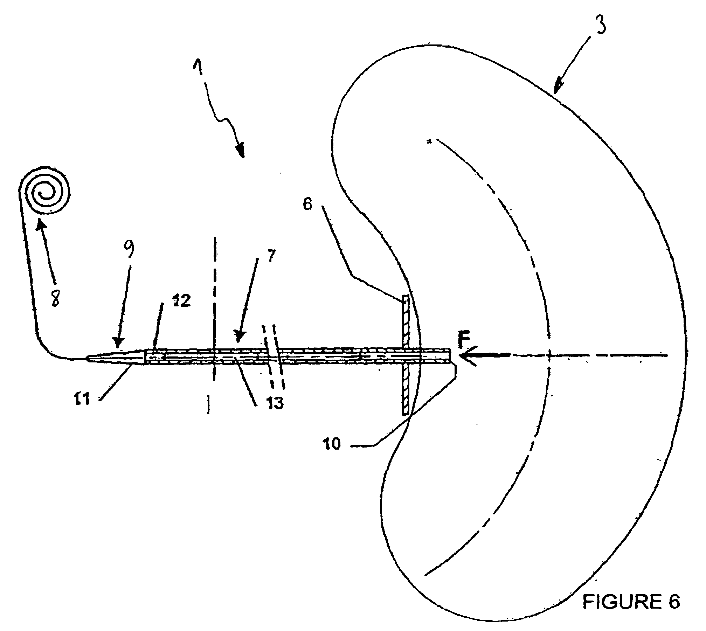

En figure 6 on a montré le dispositif intra-gastrique 1 suivant la présente invention dont le ballon ou l'enveloppe 3 présente un profil externe en forme de haricot qui est dissymétrique par rapport à l'axe horizontal du tube ou cathéter souple 7.

En effet on remarque que le ballon ou l'enveloppe 3 en forme de haricot comporte au dessus de l'axe horizontal du tube ou cathéter 7 une partie dont le volume est plus important que la partie se trouvant en dessous dudit tube ou cathéter.

Le profil dissymétrique du ballon ou enveloppe 3 par rapport à l'axe horizontal du tube ou cathéter 7 permet que ce dernier s'adapte selon le cas au profil interne de l'estomac 2.

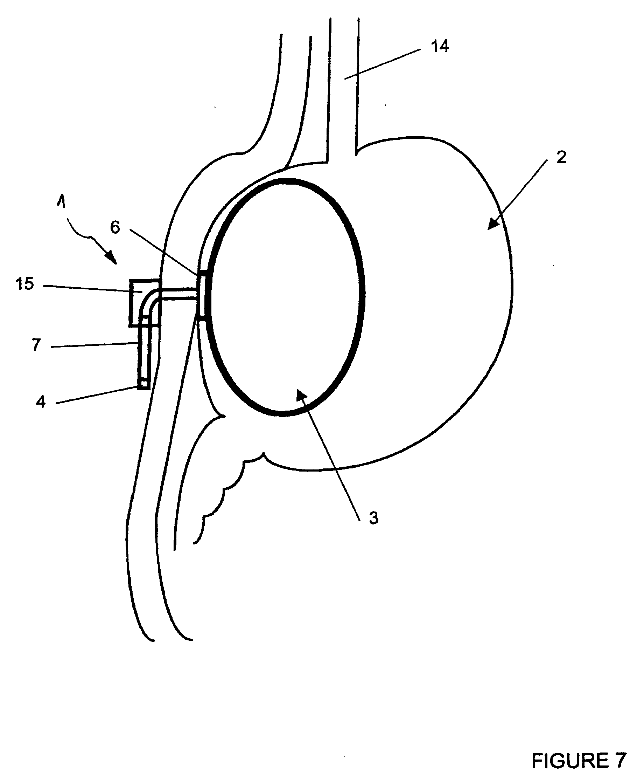

En figure 7 on a représenté le dispositif intra-gastrique 1 à l'intérieur de l'estomac 2 d'un patient. Ce dispositif est placé par voie endoscopique en passant par la bouche puis par l'oesophage 14 pour venir se positionner dans l'estomac 2.

L'opérateur procède à la récupération du tube ou cathéter 7 par voie percutanée grâce aux moyens d'accrochage 9 qui permettent d'écarter les tissus sans les déchirer.

Le tube ou cathéter 7 est fixé par un système d'attache 15 à la peau sur le ventre du patient pour permettre le maintien du dispositif intra-gastrique 1.

Ensuite, le tube ou cathéter 7 est coupé à l'extérieur du corps du patient pour éliminer l'embout 11 solidaire du fil 8 constituant les moyens d'accrochage 9, et permettre le montage d'un dispositif d'alimentation à valve anti-retour 4.

Le ballon ou enveloppe 3 est gonflé au moyen d'une seringue ou analogue, non représentée, qui s'adapte sur le dispositif d'alimentation 4 pour amener ledit ballon ou enveloppe à son volume nominal déterminé.

Dès que le volume nominal déterminé du ballon 3 est atteint, ce dernier est plaqué, par une traction externe du tube ou cathéter 7, sur la paroi interne de l'estomac 2, afin d'assurer, grâce au disque 6, une étanchéité parfaite entre l'estomac 2 et la cavité péritonéale.

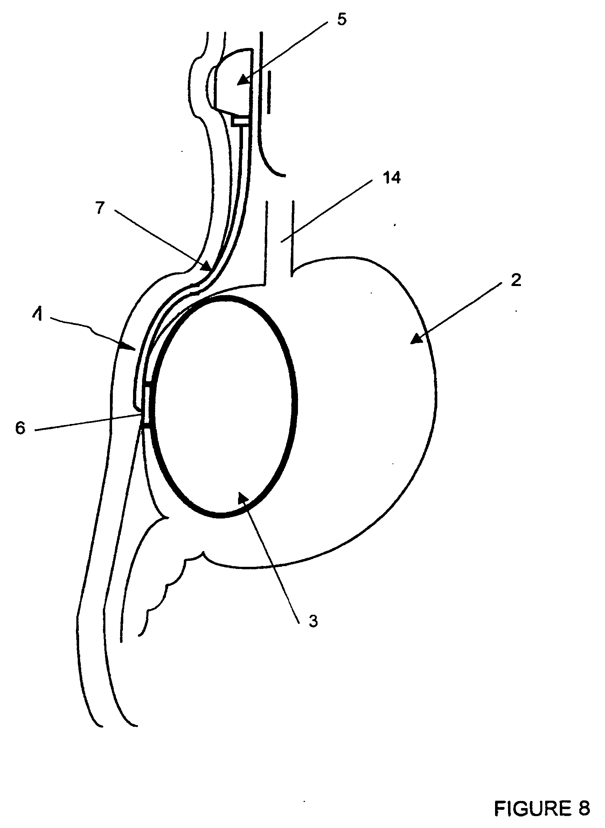

En figure 8, on a représenté le dispositif intra-gastrique 1 à l'intérieur de l'estomac 2 d'un patient. Ce dispositif est placé par voie endoscopique en passant par la bouche puis par l'oesophage 14 pour venir se positionner dans l'estomac 2.

L'opérateur procède à la récupération du tube ou cathéter 7 par voie percutanée grâce aux moyens d'accrochage 9 qui permettent d'écarter les tissus sans les déchirer.

Le tube ou cathéter 7 est fixé par un système d'attache aponévrotique qui permet le maintien du dispositif intra-gastrique 1.

Ensuite, le tube ou cathéter 7 est coupé pour éliminer l'embout 11 solidaire du fil 8, pour permettre le montage d'un dispositif d'alimentation à chambre implantable 5 qui est placé en sous cutané.

Le ballon ou enveloppe 3 est amené à son volume nominal déterminé au moyen d'une aiguille de Huber associée à une seringue, non représentée, qui est introduite dans la chambre implantable 5, commercialisée par exemple, sous la marque "DISTRICATH".

Dès que le volume nominal déterminé du ballon ou enveloppe 3 est atteint, ce dernier est plaqué, par une traction externe du tube ou cathéter 7, sur la paroi interne de l'estomac 2, afin d'assurer, grâce au disque 6, une étanchéité parfaite entre l'estomac 2 et la cavité péritonéale.

On note que le dispositif intra-gastrique 1 a pour effet de réduire le volume de l'estomac 2, donc la quantité ingérée, pour donner une sensation de satiété au patient.

On constate que le dispositif intra-gastrique 1 ne nécessite pas d'intervention chirurgicale lourde et traumatisante pour le patient.

On remarque que le dispositif intra-gastrique 1 est facilement retirable à la fin du traitement du fait de sa fixation externe ou sous cutané.

On note que le volume nominal déterminé de chaque ballon ou enveloppe 3 issu d'un jeu de dispositif intra-gastrique 1 ne vient jamais obstruer le volume maximal de l'estomac pour éviter toute occlusion de ce dernier donnant une sécurité au patient.

Il doit d'ailleurs être entendu que la description qui précède n'a été donnée qu'à titre d'exemple et qu'elle ne limite nullement le domaine de l'invention dont on ne sortirait pas en remplaçant les détails d'exécutions décrits par tout autre équivalent.

## Revendications

1. Dispositif intra-gastrique introduit par voie endoscopique dans l'estomac (2) d'un patient pour le traitement de l'obésité morbide, ledit dispositif étant issu d'un jeu de dispositif (1) comportant chacun un ballon ou enveloppe (3) présentant un volume nominal déterminé qui est raccordé de manière étanche à des moyens de connexion constitués de trois éléments, à savoir un disque (6) formant une base d'appui au ballon (3) contre la paroi interne de l'estomac (2), un tube ou cathéter souple (7) pour le raccordement du ballon (3) à un dispositif d'alimentation (4, 5), et des moyens d'accrochage (9) solidaires du tube ou cathéter (7), lesdits moyens de connexion permettant d'une part la mise en place et/ou la récupération du ballon (3), et d'autre part la fixation, soit à l'extérieur du corps du patient, soit en sous cutané du dispositif d'alimentation (4, 5) pour pouvoir amener le ballon ou l'enveloppe (3) à son volume nominal déterminé.

2. Dispositif intra-gastrique suivant la revendication 1, **caractérisé en ce que** le disque (6) est positionné autour du tube ou cathéter (7) et contre la paroi externe du ballon ou enveloppe (3).

3. Dispositif intra-gastrique suivant la revendication 2, **caractérisé en ce que** le ballon ou enveloppe (3) est fixé autour du tube ou cathéter (7) afin que ce dernier débouche à l'intérieur dudit ballon.

4. Dispositif intra-gastrique suivant la revendication 1, **caractérisé en ce que** les moyens d'accrochage (9) sont constitués d'un embout (11) à profil conique solidaire d'un fil (8).

5. Dispositif intra-gastrique suivant la revendication 4, **caractérisé en ce que** l'embout (11) comporte un tenon (12) qui pénètre à l'intérieur d'un alésage interne (13) du tube ou cathéter (7).

6. Dispositif intra-gastrique suivant la revendication 4, **caractérisé en ce que** le fil (8) présente un profil droit ou en boucle.

7. Dispositif intra-gastrique suivant la revendication 1, **caractérisé en ce que** le disque (6) est de forme circulaire.

8. Dispositif intra-gastrique suivant la revendication 1, **caractérisé en ce que** le disque (6) est de forme ovale.

9. Dispositif intra-gastrique suivant la revendication 1, **caractérisé en ce que** le ballon ou enveloppe (3) présente un profil ovale ou autre qui est symétrique par rapport à l'axe horizontal du tube ou cathéter (7).

10. Dispositif intra-gastrique suivant la revendication 1, **caractérisé en ce que** le ballon ou enveloppe (3) présente un profil ovale ou autre qui est dissymétrique par rapport à l'axe horizontal du tube ou cathéter (7).

11. Dispositif intra-gastrique suivant la revendication 1, **caractérisé en ce que** le ballon ou enveloppe (3) présente un profil en forme de haricot qui est symétrique par rapport à l'axe horizontal du tube ou cathéter (7).

12. Dispositif intra-gastrique suivant la revendication 1, **caractérisé en ce que** le ballon ou enveloppe (3) présente un profil en forme de haricot dissymétrique par rapport à l'axe horizontal du tube ou cathéter (7).

## Patentansprüche

1. Intragastrische Vorrichtung, die endoskopisch in den Magen (2) eines Patienten eingebracht wird, zur Behandlung von krankhafter Fettsucht, wobei die Vorrichtung aus einem Satz von Vorrichtungen (1) hervorgeht, die jeweils einen Ballon oder eine Hülle (3) tragen, der/die ein bestimmtes nominales Volumen aufweist, das mit Verbindungseinrichtungen dicht verbunden ist, die aus drei Bauteilen bestehen, nämlich einer Scheibe (6), die eine Auflagebasis für den Ballon (3) gegen die Innenwand des Magens (2) bildet, einem biegsamem Schlauch oder Katheter (7) zum Verbinden des Ballons (3) mit einer Versorgungsvorrichtung (4, 5) und Halteeinrichtungen (9), die mit dem Schlauch oder Katheter (7) einstückig sind, wobei die Verbindungseinrichtungen einerseits das Einführen und/oder Zurückziehen des Ballons (3) und andererseits das Fixieren der Versorgungsvorrichtung (4, 5) entweder außen am Körper des Patienten oder subkutan gestattet, damit der Ballon oder die Hülle (3) auf sein/ihr bestimmtes nominales Volumen gefüllt werden kann.

2. Intragastrische Vorrichtung nach Anspruch 1, **dadurch gekennzeichnet, dass** die Scheibe (6) um den Schlauch
oder Katheter (7) herum und gegen die Außenwand des Ballons oder der Hülle (3) angebracht ist.

3. Intragastrische Vorrichtung nach Anspruch 2, **dadurch gekennzeichnet, dass** der Ballon oder die Hülle (3) um den Schlauch oder Katheter (7) herum befestigt ist, so dass Letzterer in das Innere des Ballons mündet.

4. Intragastrische Vorrichtung nach Anspruch 1, **dadurch gekennzeichnet, dass** die Halteeinrichtungen (9) aus einem Ansatzstück (11) mit konischem Profil bestehen, das mit einem Faden (8) einstückig ist.

5. Intragastrische Vorrichtung nach Anspruch 4, **dadurch gekennzeichnet, dass** das Ansatzstück (11) einen Zapfen (12) trägt, der in das Innere einer Innenbohrung (13) des Schlauchs oder Katheters (7) eindringt.

6. Intragastrische Vorrichtung nach Anspruch 4, **dadurch gekennzeichnet, dass** der Faden (8) ein gerades oder schlaufenförmiges Profil aufweist.

7. Intragastrische Vorrichtung nach Anspruch 1, **dadurch gekennzeichnet, dass** die Scheibe (6) rund geformt ist.

8. Intragastrische Vorrichtung nach Anspruch 1, **dadurch gekennzeichnet, dass** die Scheibe (6) oval geformt ist.

9. Intragastrische Vorrichtung nach Anspruch 1, **dadurch gekennzeichnet, dass** der Ballon oder die Hülle (3) ein ovales oder anderes Profil aufweist, das in Bezug auf die Horizontalachse des Schlauchs oder Katheters (7) symmetrisch ist.

10. Intragastrische Vorrichtung nach Anspruch 1, **dadurch gekennzeichnet, dass** der Ballon oder die Hülle (3) ein ovales oder anderes Profil aufweist, das in Bezug auf die Horizontalachse des Schlauchs oder Katheters (7) asymmetrisch ist.

11. Intragastrische Vorrichtung nach Anspruch 1, **dadurch gekennzeichnet, dass** der Ballon oder die Hülle (3) ein bohnenförmiges Profil aufweist, das in Bezug auf die Horizontalachse des Schlauchs oder Katheters (7) symmetrisch ist.

12. Intragastrische Vorrichtung nach Anspruch 1, **dadurch gekennzeichnet, dass** der Ballon oder die Hülle (3) ein bohnenförmiges Profil aufweist, das in Bezug auf die Horizontalachse des Schlauchs oder Katheters (7) asymmetrisch ist.

## Claims

1. Intragastric device introduced by an endoscopy procedure into the stomach (2) of a patient for treating morbid obesity, said device being made up of a set of devices (1) each comprising a balloon or envelope (3) of defined nominal volume which is connected in a leaktight manner to connection means consisting of three elements, namely a disc (6) forming a support base for the balloon (3) against the inner wall of the stomach (2), a flexible tube or catheter (7) for connecting the balloon (3) to a supply device (4, 5), and securing means (9) integral with the tube or catheter (7), said connection means making it possible, on the one hand, to position and/or recover the balloon (3) and, on the other hand, to fix the supply device (4, 5) either outside the patient's body or subcutaneously, in order to be able to bring the balloon or envelope (3) to its defined nominal volume.

2. Intragastric device according to Claim 1, **characterized in that** the disc (6) is positioned about the tube or catheter (7) and against the outer wall of the balloon or envelope (3).

3. Intragastric device according to Claim 2, **characterized in that** the balloon or envelope (3) is fixed about the tube or catheter (7) so that the latter opens out inside said balloon.

4. Intragastric device according to Claim 1, **characterized in that** the securing means (9) consist of a joining piece (11) of conical profile integral with a filament (8).

5. Intragastric device according to Claim 4, **characterized in that** the joining piece (11) has a stub (12) which penetrates inside an internal bore (13) of the tube or catheter (7).

6. Intragastric device according to Claim 4, **characterized in that** the filament (8) has a straight or looped profile.

7. Intragastric device according to Claim 1, **characterized in that** the disc (6) is of circular shape.

8. Intragastric device according to Claim 1, **characterized in that** the disc (6) is of oval shape.

9. Intragastric device according to Claim 1, **characterized in that** the balloon or envelope (3) has an oval profile or other profile which is symmetrical with respect to the horizontal axis of the tube or catheter (7).

10. Intragastric device according to Claim 1, **characterized in that** the balloon or envelope (3) has an oval profile or other profile which is asymmetrical with respect to the horizontal axis of the tube or catheter (7).

11. Intragastric device according to Claim 1, **characterized in that** the balloon or envelope (3) has a kidney-bean-shaped profile symmetrical with respect to the horizontal axis of the tube or catheter (7).

12. Intragastric device according to Claim 1, **characterized in that** the balloon or envelope (3) has a kidney-bean-shaped profile asymmetrical with respect to the horizontal axis of the tube or catheter (7).
